# EUROPEAN PATENT APPLICATION

(11) **EP 1 484 315 A1**
(43) Date of publication of application: **08.12.2004**
(21) Application number: 03703285.1
(22) Date of filing: 10.02.2003
(51) Int. Cl.: C07C 231/20, C07C 233/47, C07C 227/20, C07C 229/34

(54) **PROCESS FOR PRODUCTION OF OPTICALLY ACTIVE BETA-PHENYLALANINE**

(30) Priority: 15.02.2002 JP 2002038758
(71) Applicant: Ajinomoto Co., Inc., Tokyo 104-8315 (JP)
(72) Inventor: NOHIRA, Hiroyuki, Saitama-shi, Saitama 336-0901 (JP)
(74) Representative: Nicholls, Kathryn Margaret
(86) International application number: PCT/JP2003/001364
(87) International publication number: WO 2003/068728

(57) **Abstract**

In the present application is disclosed a method where N-acetyl-β-phenylalanine is subjected to optical resolution by a preferential crystallization method to prepare an optically active N-acetyl-β-phenylalanine. In accordance with the method, it is possible to prepare an optically active N-acetyl-β-phenylalanine, and subsequently, an optically active β-phenylalanine in a simple and efficient manner.

## Description

### (Technical Field)

The present invention relates to methods for preparing optically active N-acetyl-β-phenylalanine. The present invention further relates to methods for the production of optically active β-phenylalanine.

In the present specification, a β-phenylalanine derivative means β-phenylalanine having a substituent on its phenyl group (a β-phenylalanine derivative in the narrow sense) , but, when there is no risk of misunderstanding in terms of the context, not only the β-phenylalanine derivative in the narrow sense but also β-phenylalanine per se may be referred to as a β-phenylalanine derivative (in the broad sense).

### (Background Art)

Optically active β-phenylalanine derivatives are known to be a raw material for receptor antagonists, enzyme inhibitors or the like, and are compounds which are useful as intermediates for pharmaceuticals, such as antithrombotic agent, etc. Known methods for producing optically active β-phenylalanine derivatives include a method in which a racemic β-phenylalanine derivative is enzymatically resolved (see, for example, J. Org. Chem., vol. 63, p.2351 (1998), as a method using penicillin acylase), a method in which the manufacture invloves asymmetric synthesis (see, for example, J. Am. Chem. Soc., vol. 116, p.10520 (1994)), etc. However, it is difficult to obtain an optically active β-phenylalanine derivative having a high optical purity in an efficient manner. On the other hand, racemic β-phenylalanine derivatives may be relatively easily produced by synthetic means (see, for example, J. Am. Chem. Soc., vol. 51, p.841 (1929)). Thus, there has been a demand for the development of a process for the optical resolution of a racemic substance for the production of optically active β-phenylalanine derivatives.

The term optical resolution is defined as follows for example. "Optical resolution - An operation by which a racemic substance is separated intoeach enantiomer, i.e. optical isomer, is called an optical resolution. In an optical resolution, there is a direct method where a racemic substance is directly resolved into optical isomers and a method where a racemic substance is made to react with an optically active reagent (optical resolving agent) to give diastereomers, resolution into each diastereomer is conducted utilizing the difference in physical property between the diastereomers and the optically active reagent is again removed to give an optically active substance. Representative means in the direct method are a preferential crystallization where crystals of an optical active substance (crystal seeds) are added to a saturated solution of a racemic substance to promote the crystallization whereupon an optical active substance is prepared (preferential crystallization method) and a column chromatography where an optically active stationary phase is used. When a racemic substance is an acid for example, a typical method for the preparation of a diastereomer is that a diastereomer salt with an optically active base such as alkaloid (e.g., quinine and brucine) is prepared, recrystallization is conducted to separate it as a pure desired diastereomer salt and the resulting salt is decomposed with an acid or an alkali to give an optically active substance." ("Kagaku Jiten" (Encyclopedic Dictionary of Chemistry), page 458, published by Tokyo Kagaku Dojin in 1994).

Of the optical resolution methods mentioned, the process for the production of an optically active N-acetyl-β-phenylalanine derivative according to the present invention, as described in detail below, is in accordance with the preferential crystallization method

As mentioned above, the preferential crystallization method has been known as one of the means for optical resolution (see, for example, Chem. Ind., vol. 53, p.10 (1934)). To be more specific, it is a method in which seed crystals of one of the optically active substances are inoculated to a racemic substance in a supersaturated state in a solution to preferentially crystalize only the same kind of the active substances to thereby effect an optical resolution. However, the use of the preferential crystallization method is limited to compounds in which crystals of the racemic substance form a racemic mixture. On the other hand, when crystals of a racemic substance form a racemic compound, they are unable to be directly subjected to an optical resolution by a preferential crystallization method. In order to find a compound which forms a racemic mixture and also to clarify that the said compound is able to be efficiently subjected to an optical resolution by means of the preferential crystallization method, it is necessary to repeat trials-and-errors by means of actual tests for various compounds. In addition, in the preferential crystallization method in which crystallization is carried out after inoculation of seed crystals of one of the optically active substances, it is necessary that the crystals are grown while preventing the natural crystallization of the other optically active substance. Thus, an efficient optical resolution is not possible unless stability upon supersaturation of the other optically active substance which is different from the desired active substance is high, and the current situation is that the ideal case where the preferential crystallization method is industrially applicable in the actual state is quite rare.

For example, N-acetyl-DL-α-phenylalanine is not a racemic mixture but is a racemic compound, and it is known that the compound is unable to be subjected to an optical resolution utilizing a preferential crystallization method and that application of a preferential crystallization method is possible only when an amine salt is formed with a specific amine which forms a racemic mixture (Nippon Kagaku Kaishi, No. 8, p.1189 (1983)). Even in the case in which a salt is formed with a specific compound to form a racemic mixture is found, it is still necessary that the crystals of the resulting salt are subjected to a double decomposition and the part of the above-mentioned specific compound is removed.

### (Disclosure of the Invention)

### [Problems to be Solved by the Invention]

Under the above-mentioned background art, it is an object of the present invention to provide industrially advantageous processes for the production of optically active N-acetyl-β-phenylalanine, and consequently, optically active β-phenylalanine, which is prepared by deacetylation of thereof, both being useful as intermediates for pharmaceuticals, etc.

### [Means for Solving the Problems]

In order to achieve the above-mentioned obj ect, the present inventor has carried out intensive studies and found that crystals of N-acetyl-β-phenylalanine, in which the amino group of β-phenylalanine is acetylated, form a racemic mixture and are able to be optically resolved in an efficient manner by means of the preferential crystallization method. They have achieved the present invention on these findings.

Thus, the present invention comprises the following.
[1] A process for the production of an optically active N-acetyl-β-phenylalanine, comprises subjecting N-acetyl-β-phenylalanine to optical resolution by a preferential crystallization method.
[2] A process for the production of an optically active N-acetyl-β-phenylalanine, comprises acetylating the amino group of β-phenylalanine, to obtain N-acetyl-β-phenylalanine, and subjecting the resulting N-acetyl-β-phenylalanine to optical resolution by a preferential crystallization method.
[3] The process for the production of an optically active N-acetyl-β-phenylalanine according to [1] or [2], wherein, at the stage of the preferential crystallization, a salt of said N-acetyl-β-phenylalanine is made to coexist.
[4] The process for the production of an optically active N-acetyl-β-phenylalanine according to [3], wherein said salt made to be coexistent is a 1-amino-2-propanol salt of said N-acetyl-β-phenylalanine.
[5] A process for the production of an optically active β-phenylalanine, comprising subjecting the optically active N-acetyl-β-phenylalanine produced according to the producing process of any of [1] to [4] above to a deacetylation reaction.

### [Embodiments of the Invention]

The present invention will now be illustrated in more detail below.

There is no particular limitation for the method of acetylating the β-phenylalanine; any method which is known to persons skilled in the art may be used. For example, acetic acid may be used as an acetylating agent and made to react with β-phenylalanine to give the desired N-acetylated product.

In accordance with the present invention, N-acetyl-β-phenylalanine is optically resolved by means of a preferential crystallization method. Thus, when seed crystals of (+)-N-acetyl-β-phenylalanine or (-)-N-acetyl-β-phenylalanine are added to a solution of (±)-N-acetyl-β-phenylalanine followed by crystallization, an optically active N-acetyl-β-phenylalanine of the same type as that of the seed crystals is preferentially crystallized.

To be more specific, (±)-N-acetyl-β-phenylalanine is firstly dissolved in an appropriate solvent to prepare a supersaturated solution. The supersaturated solution as such may be prepared, for example, dissolving (±) -N-acetyl-β-phenylalanine in a solvent at a high temperature and then cooling or concentrating the resulting solution. Examples of preferred solvents include water and organic solvents such as methanol, ethanol, isopropanol, methyl ethyl ketone, acetone, ethyl acetate and the like, and among them, water is particularly preferred. Although concentration of (±)-N-acetyl-β-phenylalanine in the supersaturated solution varies depending upon the solvent used therefor, the crystallization temperature, etc., it is usually set within a range of 3 to 50% by weight. Incidentally, it is not always necessary that N-acetyl-β-phenylalanine is a racemic substance, and it goes without saying that a substance in which one of the optically active compounds is contained in a greater amount than the other optically active compound may also be subjected to the method of the present invention.

When seed crystals of an optically active N-acetyl-β-phenylalanine are added, there is no particular limitation for the amount to be added so long as the crystals can be deposited. Usually however, the seed crystals are added within a range of 0.01 to 10% to the weight of (±)-N-acetyl-β-phenylalanine dissolved therein.

It goes without saying that, when seed crystals of (+)-N-acetyl-β-phenylalanine are added, (+)-N-acetyl-β-phenylalanine is crystallized preferentially while, when seed crystals of (-)-N-acetyl-β-phenylalanine are added, (-)-N-acetyl-β-phenylalanine is crystallized preferentially. In the method of the present invention, crystallization may be carried out from any of the optically active substances. The crystallization may be carried out with the supersaturated solution being allowed to stand or with stirring.

The crystallized optically active crystals may be separated by any known method such as filtration, and (±)-N-acetyl-β-phenylalanine may be added to the mother liquor after separation of the crystals to prepare a supersaturated solution again. In the mother liquor, the enantiomer of N-acetyl-β-phenylalanine other than the separated crystals (antipode) exists in an excess, and, in the next step, seed crystals of the isomer (antipode) which exists in excess are added, followed by subjecting to crystallization in a similar manner, whereupon it is possible to prepare crystals of the antipode to the already-separated optically active substance. When those operations are repeated, it is possible to prepare crystals of (+)-N-acetyl-β-phenylalanine and those of (-) -N-acetyl-β-phenylalanine alternately whereupon an optical resolution of (±)-N-acetyl-β-phenylalanine is efficiently carried out.

With regard to the resulting crystals, those having the same optical activity may be combined and recrystallized from an appropriate solvent such as ethanol, whereby the optical purity may be further enhanced.

By conducting the preferential crystallization by formation of a salt of N-acetyl-β-phenylalanine with an appropriate acid or base, it is possible to stabilize the supersaturated state of the supersaturated solution and to carry out the optical resolution more efficiently when the salt is made to be coexistent in the supersaturated solution. Examples of the acid for forming such a salt coexistent therein are hydrochloric acid, sulfuric acid, and the like, while examples of the base therefor are ammonia, aminoethanol, 1-amino-2-propanol, and the like. Among the bases, 1-amino-2-propanol is particularly preferred, and it is preferred that the salt of N-acetyl-β-phenylalanine therewith is made to be coesistent.

Optically active β-phenylalanine may be prepared by deacetylating the optically active N-acetyl-β-phenylalanine prepared by such an optical resolution by any deacetylation reaction known to persons skilled in the art, such as a deacetylation using an acid.

### (Best Mode for Carrying Out the Invention)

The present invention will now be illustrated in more detail by way of the following Example, but the present invention is not limited to the Example.

### <Example 1>

(±)-N-Acetyl-β-phenylalanine (1100 mg) (5.31 mmol) and 400 mg (5.33 mmol) of (±)-1-amino-2-propanol were added to a 30-ml Erlenmeyer flask and dissolved in 4.0 ml of water. To the resulting solution were further added 882 mg (4.26 mmol) of (±)-N-acetyl-β-phenylalanine followed by dissolving with heating. The resulting solution was allowed to cool so that the temperature of the solution was lowered to room temperature, then 10 mg of seed crystals of (+)-N-acetyl-β-phenylalanine suspended in 0.4 ml of water were added thereto, the mixture was allowed to stand for 7 hours, and the crystals of (+)-N-acetyl-β-phenylalanine which separated therefrom were collected by filtration.

(±)-N-Acetyl-β-phenylalanine in the same amount as the crystallized crystals was added to the mother liquor and dissolved with heating, and seed crystals of (-)-N-acetyl-β-phenylalanine were added, followed by crystallizing in the same manner whereupon crystals of (-)-N-acetyl-β-phenylalanine were obtained. Those operations were repeated six times in total (see the following Table 1), and the resulting crystals which exhibited the same optical activity were combined and recrystallized from 99% ethanol to give crystals of (+)-N-acetyl-β-phenylalanine and those of (-)-N-acetyl-β-phenylalanine (see the following Table 2).

**Table 1**

| Crystallization No. | Yield (mg) | Melting Point (°C) | Angle of Rotation (°) |
|---|---|---|---|
| 1 | 110 | 189-192 | +73.0 |
| 2 | 121 | 166-181 | -57.2 |
| 3 | 71 | 186-190 | +70.4 |
| 4 | 464 | 182-189 | -69.7 |
| 5 | 682 | 184-192 | +78.5 |
| 6 | 686 | 189-191 | -80.7 |

**Table 2**

| | Yield (mg) | Melting Point (°C) | Angle of Rotation (°) |
|---|---|---|---|
| (+)-N-Acetyl-β-phenylalanine | 659 | 192-193 | +91.4 |
| (-)-N-Acetyl-β-phenylalanine | 872 | 193-194 | -89.2 |

(Note 1) With regard to (+)-N-acetyl-β-phenylalanine, the data are for the crystals prepared by combination of crystals of Nos. 1, 3 and 5, in Table 1, followed by recrystallizing from 3.5 ml of 99% ethanol.

(Note 2) With regard to (-)-N-acetyl-β-phenylalanine, the data are for the crystals prepared by combination of crystals of Nos. 2, 4 and 6, in Table 1, followed by recrystallizing from 5.4 ml of 99% ethanol.

(Note 3) The angle of rotation is the value at c = 1.0, methanol. Incidentally, the value of the angle of rotation for (+)-N-acetyl-β-phenylalanine in the literatures is [α]D = +84.9° (c = 0.6, methanol) and that for (-)-N-acetyl-β-phenylalanine is [α]D = -84.5° (c = 1.0, methanol).

### (Industrial Applicability)

In accordance with the method of the present invention, it is now possible to prepare an optically active N-acetyl-β-phenylalanine and also to prepare an optically active β-phenylalanine by deacetylation of the above in a simple manner and in a high yield.

## Claims

1. A process for the production of an optically active N-acetyl-β-phenylalanine, comprises subjecting N-acetyl-β-phenylalanine to optical resolution by a preferential crystallization method.

2. A process for the production of an optically active N-acetyl-β-phenylalanine, comprises acetylating the amino group of β-phenylalanine, to obtain N-acetyl-β-phenylalanine, and subjecting the resulting N-acetyl-β-phenylalanine to optical resolution by a preferential crystallization method.

3. The process for the production of an optically active N-acetyl-β-phenylalanine according to Claim 1 or 2, wherein, at the stage of the preferential crystallization, a salt of said N-acetyl-β-phenylalanine is made to coexist.

4. The process for the production of an optically active N-acetyl-β-phenylalanine according to Claim 3, wherein said salt made to be coexistent is a 1-amino-2-propanol salt of said N-acetyl-β-phenylalanine.

5. A process for the production of an optically active β-phenylalanine, comprising subjecting the optically active N-acetyl-β-phenylalanine produced according to the producing process of any one of Claim 1 to 4 to a deacetylation reaction.
